Europäisches Patentamt

European Patent Office

Office européen des brevets

Veröffentlichungsnummer: **0 275 460**
A1

# EUROPÄISCHE PATENTANMELDUNG

(21) Anmeldenummer: 87118243.2

(22) Anmeldetag: 09.12.87

(51) Int. Cl.⁴ **A61B 17/22** , G10K 9/12 , G10K 11/30 , A61B 8/00

(30) Priorität: 22.12.86 DE 3643971

(43) Veröffentlichungstag der Anmeldung:
27.07.88 Patentblatt 88/30

(84) Benannte Vertragsstaaten:
DE FR GB NL

(71) Anmelder: Siemens Aktiengesellschaft Berlin und München
Wittelsbacherplatz 2
D-8000 München 2(DE)

(72) Erfinder: Grasser, Franz, Dipl.-Ing. (FH)
Neuwiesenstrasse 27
D-8557 Eggolsheim(DE)

(54) Stosswellenkopf zum berührungslosen Zertrümmern von Konkrementen.

(57) Die Erfindung betrifft einen Stoßwellenkopf (1) zum berührungslosen Zertrümmern eines im Körper eines Lebewesens befindlichen Konkrementes (19) mit einem im wesentlichen in einer Ebene Stoßwellen erzeugenden Stoßwellengenerator (3) und einer ihm zugeordneten Linsenanordnung (9), die die Stoßwelle auf einen Fokusbereich im Zielgebiet fokussiert. Die Linsenanordnung (9) liegt verstellbar an einem Anschlag (12) derart an, daß sie bei Anstoßen des Patienten (17) in Richtung auf den Stoßwellengenerator (3) nachgibt.

## Stoßwellenkopf zum berührungslosen Zertrümmern von Konkrementen

Die Erfindung betrifft einen Stoßwellenkopf zum berührungslosen Zertrümmern eines im Körper eines Lebewesens befindlichen Konkrementes mit einem im wesentlichen in einer Ebene Stoßwellen erzeugenden Stoßwellengenerator und einer ihm zugeordneten Linsenanordnung, die die Stoßwelle auf einen Fokusbereich im Zielgebiet fokussiert. Stoßwellenköpfe dieser Art werden in der Medizin beispielsweise zum Zerstören von Steinen in der Niere des Menschen eingesetzt. Sie sind besonders vorteilhaft, da sie jeglichen Eingriff in den Körper vermeiden. Es ist nicht notwendig, operativ vorzugehen.

In der DE-OS 33 28 051 ist ein derartiger Stoßwellenkopf beschrieben, der ein Stoßwellenrohr, bestehend aus einem Mantel, einer Flachspule und einer durch eine Isolierfolie getrennte Kupfermembran, aufweist. In dem Stoßwellenrohr ist eine akustische Sammellinse angeordnet, die die von der Membran erzeugten ebenen Stoßwellen in einem Brennpunkt fokussiert. Zur Ankopplung des Stoßwellenrohres an den Patienten ist die der Membran gegenüberliegende Öffnung des Stoßwellenrohres mit einem Sack abgeschlossen, der wie das gesamte Stoßwellenrohr mit einem Koppelmedium gefüllt ist. Zur Ankopplung wird das Stoßwellenrohr so lange auf den Patienten zugefahren, bis daß das zu zerstörende Konkrement sich im Brennpunkt der Linsenanordnung befindet. Hierbei legt sich der mit der Koppelflüssigkeit gefüllte Sack an der Oberfläche des Patienten an, so daß gewährleistet ist, daß die Stoßwellen immer innerhalb der Flüssigkeit verlaufen.

Bei korpulenten Patienten tritt dabei das Problem auf, daß das zu zerstörende Konkrement so weit innerhalb des Körpers des Patienten liegt, daß der Fokusabstand der Linsenanordnung nicht mehr ausreicht. Dadurch berührt das Stoßwellenrohr bzw. die Linsenanordnung den Patienten und bei Fortsetzung des Ankoppelvorganges schieben sie den Patienten sogar zur Seite. Dadurch wird das Konkrement aus dem Fokus bewegt, so daß eine Behandlung nicht erfolgen kann.

Die Erfindung geht von der Aufgabe aus, einen Stoßwellenkopf der eingangs genannten Art derart auszubilden, daß der Einsatzbereich des Stoßwellenkopfes erweitert wird, so daß auch korpulente Patienten behandelt werden können.

Die Aufgabe wird erfindungsgemäß dadurch gelöst, daß die Linsenanordnung verstellbar an einem Anschlag derart anliegt, daß sie bei Anstoßen des Patienten in Richtung auf den Stoßwellengenerator nachgibt. Dadurch wird erreicht, daß während des Ankoppelvorganges nicht der Patient bewegt wird, so daß das Konkrement aus dem Fokusbereich herauswandert, sondern die Linsenanordnung nachgibt, wobei der Fokusbereich auf den Stoßwellenkopf zurückbewegt wird. Das Konkrement liegt aber weiterhin innerhalb des Fokusbereiches, so daß es bei Auslösen von Stoßwellen zerstört werden kann.

Es hat sich als vorteilhaft erwiesen, wenn die Linsenanordnung durch eine Feder gegen den Anschlag gedrückt wird. Ein Auswandern des Konkrementes aus dem Fokusbereich wird verhindert, wenn der Stellweg für die Linsenanordnung durch einen zweiten Anschlag begrenzt ist und wenige Zentimeter beträgt. Dieser Stellweg entspricht im wesentlichen dem Abstand des Fokus zum Fokusbereich. Eine Anzeige des Auswanderns der Linsenanordnung sowie des Auswanderns des Konkrementes aus dem Fokusbereich kann erfolgen, wenn die Anschläge mit Endschaltern versehen sind.

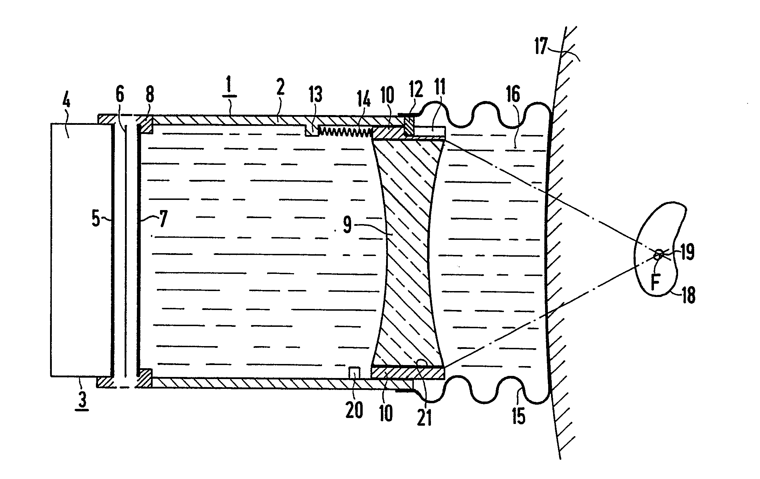

Die Erfindung ist nachfolgend anhand eines in der Zeichnung dargestellten Ausführungsbeispieles näher erläutert.

In der Figur ist ein Stoßwellenkopf 1 mit einem mit einer Flüssigkeit, z.B. Wasser, gefüllten Mantel 2 dargestellt, dessen eine Öffnung durch einen Stoßwellengenerator 3, bestehend aus einem Spulenträger 4, einer Flachspule 5, einer Isolierfolie 6 und einer Membran 7, die durch einen Rückhaltering 8 eng aneinander gepreßt zusammengehalten werden, abgeschlossen wird. Aus Gründen einer besseren Übersicht sind die Flachspule 5, die Isolierfolie 6 und die Membran 7 jeweils im Abstand voneinander gezeichnet.

Die andere Öffnung des Mantels 2 des Stoßwellenkopfes 1 wird durch eine Linsenanordnung 9 mit der Funktion einer akustischen Sammellinse abgedeckt, die von einer Halterung 10 gefaßt ist. Die Halterung 10 weist auf ihrem Umfang mehrere Nuten 11 auf, die sich etwa von der Mitte der Halterung 10 an von dem Stoßwellengenerator 3 weg erstrecken. In die Nuten 11 greifen am Mantel 2 angeordnete Nasen als erste Anschläge 12 ein. Durch mehrere an weiteren Nasen 13 sich abstützende Druckfedern 14 werden die Halterung 10 und die Linsenanordnung 9 gegen die ersten Anschläge 12 gepreßt. Der Mantel 2 des Stoßwellenkopfes 1 und die Linsenanordnung 9 werden von einem Sack 15 abgedeckt, der mit Koppelflüssigkeit 16 gefüllt ist. Im angekoppelten Zustand liegt der Sack 15 an der Haut eines Patienten 17 an. Dann befindet sich ein in einer Niere 18 angeordnetes Konkrement 19 in dem Brennpunkt F der Linsenanordnung 9, so daß es durch vom Stoßwellengenerator 3 erzeugte Stoßwellen zerstört kann.

Ist nun der Patient 17 derart korpulent, daß der Abstand Haut zur Niere 18 und damit des Konkrementes 19 größer ist als der Fokusabstand, stößt die Oberfläche des Patienten 17 an der Linsenanordnung 9 an. Nun wird während des Ankoppelvorganges die Linsenanordnung 9 gegen die Kraft der Federn 14 in Richtung auf den Stoßwellengenerator 3 verschoben. Dies kann so lange erfolgen, bis daß das Konkrement 19 noch innerhalb des Fokusbereiches liegt. Da dieser Fokusbereich in Längsrichtung des Stoßwellenkopfes 1 eine Abmessung von einigen Zentimetern aufweist, läßt sich die Linsenanordnung 9 in gleichem Maße verschieben, ohne daß das Konktrement 19 aus dem Fokusbereich herauswandert. Deshalb sind auf dem Umfang des Mantels 2 des Stoßwellenkopfes 1 zweite Anschläge 20 vorgesehen, gegen die die Halterung 10 der Linsenanordnung 9 als Begrenzung ihres Stellweges stößt.

Die Linsenanordnung 9 ist mit einer Feinregulierung 20 in Richtung auf den Fokus F relativ zur Halterung 10 und damit zum Mantel 2 des Stoßwellenkopfes 1 verschiebbar.

An den Anschlägen 12 und 20 können Endschalter angeordnet sein, So wird beispielsweise der Endschalter an dem ersten Anschlag 12 bei Verschieben der Linsenanordnung 9 betätigt, so daß dadurch der Untersuchungsperson angezeigt werden kann, daß sich die Linsenanordnung 9 aus ihrer Endlage bewegt hat und damit bei vollständiger Ankopplung das Konkrement sich nicht mehr innerhalb des Fokus, d.h. innerhalb des Maximums, befindet, sondern nur noch innerhalb des Fokusbereiches liegt. Wird die Linsenanordnung 9 dagegen gegen den zweiten Anschlag 20 bewegt, so kann zum einen der Ankoppelvorgang sofort unterbrochen werden, während gleichzeitig beispielsweise auf dem Monitor der Untersuchungsperson angezeigt wird, daß eine Ankopplung nicht erfolgen kann, da das Konkrement außerhalb des Fokusbereiches liegt.

## Ansprüche

1. Stoßwellenkopf (1) zum berührungslosen Zertrümmern eines im Körper eines Lebewesens befindlichen Konkrementes (19) mit einem im wesentlichen in einer Ebene Stoßwellen erzeugenden Stoßwellengenerator (3) und einer ihm zugeordneten Linsenanordnung (9), die die Stoßwelle auf einen Fokusbereich im Zielgebiet fokussiert, **dadurch gekennzeichnet,** daß die Linsenanordnung (9) verstellbar an einem Anschlag (12) derart anliegt, daß sie bei Anstoßen des Patienten (17) in Richtung auf den Stoßwellengenerator (3) nachgibt.

2. Stoßwellenkopf (1) nach Anspruch 1, **dadurch gekennzeichnet,** daß die Linsenanordnung (9) durch eine Feder (14) gegen den Anschlag (12) gedrückt wird.

3. Stoßwellenkopf nach Anspruch 1 oder 2, **dadurch gekennzeichnet,** daß der Stellweg für die Linsenanordnung (9) durch einen zweiten Anschlag (20) begrenzt ist und wenige Zentimeter beträgt.

4. Stoßwellenkopf nach Anspruch 3, **dadurch gekennzeichnet,** daß die Anschläge (12, 20) mit Endschaltern versehen sind.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 133 665  (SIEMENS) <br> * Seite 6, Zeilen 33-37; Zusammenfassung * <br> --- | 1,3 | A 61 B   17/22 <br> G 10 K    9/12 <br> G 10 K   11/30 <br> A 61 B    8/00 |
| A | DE-A-3 444 421  (DORNIER) <br> * Insgesamt * <br> --- | 1 | |
| A | WO-A-8 204 157  (BATTELLE) <br> * Zusammenfassung * <br> --- | 1 | |
| A | US-A-4 194 510  (A.P. PROUDIAN) <br> * Spalte 3, Zeilen 43-61; Figuren * <br> --- | 1 | |
| A | US-A-3 800 276  (G.T. RISHELL) <br> * Spalte 3, Zeile 58 - Spalte 4, Zeile 28; Figur 3 * <br> ----- | 1 | |

RECHERCHIERTE
SACHGEBIETE (Int. Cl.4)

A 61 B
A 61 F
G 10 K

Der vorliegende Recherchenbericht wurde für alle Patentansprüche erstellt

| Recherchenort | Abschlußdatum der Recherche | Prüfer |
|---|---|---|
| DEN HAAG | 03-03-1988 | WOLF C.H.S. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
................................................
& : Mitglied der gleichen Patentfamilie, übereinstimmendes
    Dokument

EPO FORM 1503 03.82 (P0401)